# FASCICULE DE BREVET EUROPEEN

(11) **EP 1 037 564 B1**
(45) Date de publication et mention de la délivrance du brevet: **09.04.2003**
(21) Numéro de dépôt: 98958974.2
(22) Date de dépôt: 08.12.1998
(51) Int. Cl.: A61B 17/70

(54) **CONNECTEUR POUR DISPOSITIF D'OSTEOSYNTHESE RACHIDIENNE**
VERBINDER FÜR VORRICHTUNG ZUR OSTEOSYNTHESE DER WIRBELSÄULE
CONNECTOR FOR BACKBONE OSTEOSYNTHESIS DEVICE

(30) Priorité: 09.12.1997 FR 9715540
(43) Date de publication de la demande: 27.09.2000
(73) Titulaire: Dimso Distribution Medicale du Sud Ouest, 33610 Cestas (FR)
(72) Inventeur: LE COUEDIC, Régis, F-33600 Pessac (FR); LAVERGNE, Michel, F-33700 Merignac (FR)
(74) Mandataire: Le Forestier, Eric
(86) Numéro de dépôt international: FR9802651
(87) Numéro de publication internationale: WO99029248

(56) Documents cités:
- EP-A- 0 778 007
- EP-A- 0 813 845

## Description

L'invention concerne les dispositifs d'ostéosynthèse.

On connaît d'après le document EP-0 778 007-A1 un dispositif d'ostéosynthèse rachidienne comportant une tige, une traverse, un connecteur, une bague et un écrou. Le connecteur comporte deux mors adaptés à serrer la tige. Il présente un logement pour la réception de la traverse s'étendant suivant une direction perpendiculaire à la tige reçue entre les mors. Le connecteur présente un filetage pour l'engagement de l'écrou. Pour monter le dispositif, on clipse les mors sur la tige, puis on enfile la bague sur le connecteur de sorte qu'elle vient en appui sur les mors. On introduit la traverse dans le logement. On visse enfin l'écrou sur le connecteur. L'écrou sollicite la traverse elle-même en appui sur la bague. Grâce à des faces inclinées assurant le contact entre la bague et les mors, la bague sollicite à son tour les mors en direction l'un de l'autre pour le serrage de la tige. Un tel dispositif permet de relier rigidement une tige longitudinale à une traverse, et plus complètement de relier par une traverse commune deux tiges longitudinales s'étendant généralement parallèlement le long de la colonne et reliées chacune à la colonne. Toutefois, ce dispositif comprend un nombre élevé de pièces. Son installation nécessite donc un grand nombre d'opérations et son montage est relativement compliqué.

Un but de l'invention est de fournir un dispositif dont le montage soit plus rapide et plus simple.

En vue de la réalisation de ce but, on prévoit selon l'invention un connecteur pour dispositif d'ostéosynthèse du rachis, comportant deux mors et présentant un logement adapté à recevoir une tige prenant appui sur les mors, le connecteur étant agencé de sorte qu'une sollicitation sur la tige reçue dans le logement, en direction des mors, sollicite les mors en direction l'un de l'autre, dans lequel le connecteur est d'une seule pièce.

Ainsi, le dispositif d'ostéosynthèse comporte un nombre réduit de pièces. Le nombre d'opérations d'assemblage à effectuer préalablement à l'intervention chirurgicale ou durant celle-ci est donc réduit et le montage du dispositif est simplifié.

Avantageusement, le connecteur comporte au moins une jonction contiguë aux mors, la jonction ayant une largeur inférieure à une largeur du connecteur au niveau des mors.

Ainsi, on accroît la flexibilité élastique des mors par rapport au reste du connecteur sans avoir à donner de grandes dimensions au connecteur pour atteindre une flexibilité équivalente.

Avantageusement, la jonction présente une fente s'étendant à partir des mors en direction opposée à ceux-ci.

Ainsi, on augmente encore la flexibilité des mors.

Avantageusement, le connecteur comporte une tête reliée aux mors par la jonction, la largeur de la jonction étant inférieure à une largeur de la tête.

Ainsi, si la tête est destinée à recevoir un organe de serrage, les dimensions de la tête peuvent être importantes pour faciliter sa fabrication.

Avantageusement, le connecteur est agencé de sorte que la tige reçue dans le logement est en appui sur une zone des mors distante de la jonction.

Ainsi, on augmente le bras de levier exercé par la tige transversale sur les mors, ce qui accroît le serrage des mors sur la tige longitudinale.

Avantageusement, le connecteur est agencé de sorte que la tige reçue dans le logement est en appui sur un bord des mors distant de la jonction.

Avantageusement, les mors présentent chacun une face plane adaptée à s'étendre en regard de la tige reçue dans le logement, cette face étant inclinée vers l'intérieur du connecteur.

Avantageusement, le connecteur présente une face filetée, le logement s'étendant entre les mors et la face filetée.

Avantageusement, la face filetée est formée par un conduit s'étendant dans le connecteur.

Ainsi, on évite que le filet soit exposé vers l'extérieur et ne provoque des blessures du corps arthrodésé.

On prévoit également selon l'invention un dispositif d'ostéosynthèse comportant des première et deuxième tiges, un organe fileté et un connecteur comportant deux mors adaptés à serrer la première tige, le connecteur présentant une face filetée et un logement s'étendant entre les mors et la face filetée et adapté à recevoir la deuxième tige prenant appui sur les mors, le connecteur étant agencé de sorte qu'une sollicitation par l'organe fileté coopérant avec la face filetée, sur la deuxième tige reçue dans le logement, en direction des mors, sollicite les mors en direction l'un de l'autre pour le serrage de la première tige, dans lequel le connecteur est d'une seule pièce.

Avantageusement, les mors sont adaptés à être clipsés sur la première tige.

Avantageusement, les mors sont adaptés à serrer la première tige en l'absence d'une sollicitation sur les mors.

Avantageusement, les mors sont configurés de sorte que la première tige s'étend en saillie des mors suivant une direction radiale à la première tige, lorsque la première tige est reçue entre les mors.

Ainsi, il est inutile de ménager un espace important entre la tige et le corps arthrodésé. Le dispositif peut donc être positionné très près du corps.

Avantageusement, la première tige forme une liaison intervertébrale le long d'un rachis, et la deuxième tige forme une union transversale entre la première tige et une autre tige formant une liaison intervertébrale.

Avantageusement, le connecteur est agencé de sorte que la deuxième tige peut être reçue dans le logement en ayant une position angulaire variable par rapport à une direction perpendiculaire à la première tige.

Ainsi, on peut adapter à volonté la position relative des deux tiges à la configuration du dispositif monté sur le patient.

D'autres caractéristiques et avantages de l'invention apparaîtront encore dans la description suivante d'un mode préféré de réalisation donné à titre d'exemple non limitatif. Aux dessins annexés :
- les figures 1 à 3 sont des vues respectivement en perspective, de face et de côté d'un connecteur selon l'invention ;
- la figure 4 est une vue en perspective d'un dispositif d'ostéosynthèse selon l'invention à l'état monté, comprenant deux connecteurs de la figure 1 ;
- la figure 5 est une vue à plus grande échelle d'une partie de la figure 4 ; et
- les figures 6 et 7 sont des vues respectivement de face et de dessus du dispositif de la figure 5.

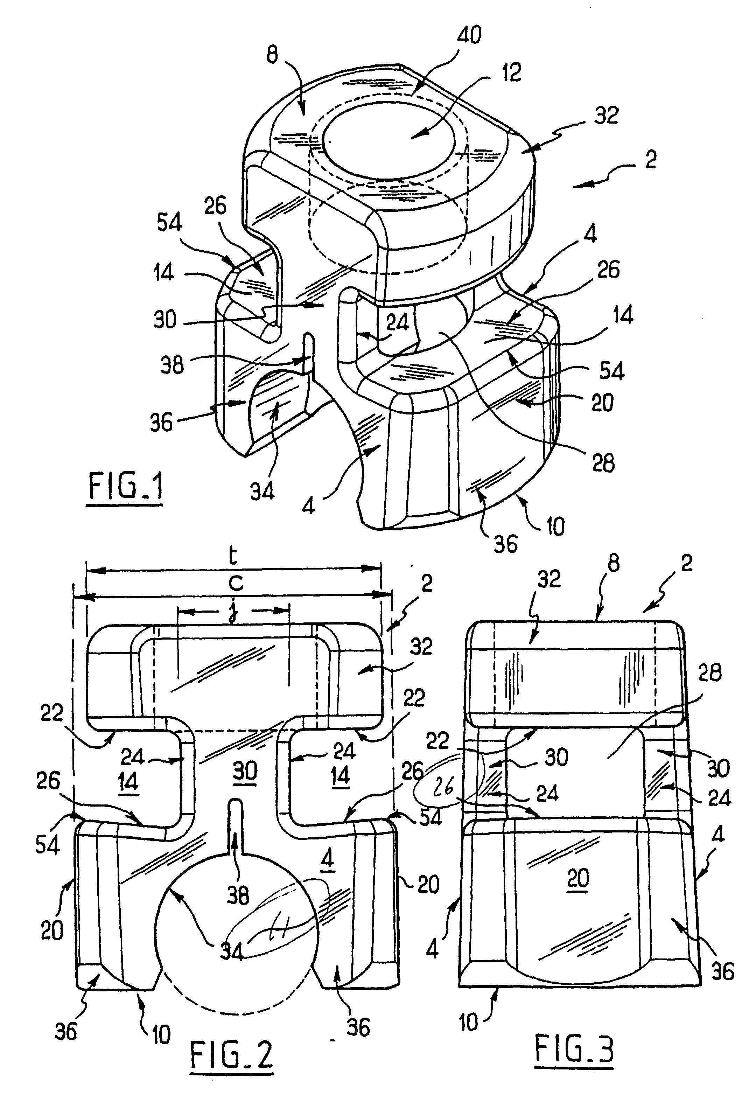

En référence aux figures 1 à 3, le connecteur 2 selon le présent mode de réalisation de l'invention a une forme générale de parallélépipède rectangle présentant les découpes qui vont être détaillées plus loin et, au niveau de ses arêtes, des arrondis évitant tout risque de blessure avec le corps arthrodésé.

Le connecteur présente deux faces planes verticales avant et arrière 4 sensiblement parallèles entre elles et s'étendant en continuité sur toute la hauteur du connecteur. Il présente également deux faces planes horizontales supérieure et inférieure 8, 10 parallèles entre elles et sensiblement perpendiculaires aux faces verticales 4 précitées. Le connecteur présente un conduit 12 généralement cylindrique s'étendant parallèlement aux faces avant et arrière 4, à mi-distance de celles-ci, et perpendiculairement aux faces supérieure et inférieure 8, 10 dans lesquelles il débouche. Le connecteur présente une symétrie axiale suivant l'axe du conduit 12.

Le connecteur présente deux échancrures 14 s'étendant généralement parallèlement aux faces supérieure et inférieure 8, 10 à partir de deux faces latérales 20 du connecteur perpendiculaires aux faces avant et arrière 4. Chaque échancrure 14 a un profil général en « U » et est délimitée par une face supérieure 22 parallèle à la face supérieure 8 du connecteur, une face de fond 24 parallèle aux faces latérales 20 du connecteur, et une face inférieure 26 s'étendant en regard de la face supérieure 22 en étant perpendiculaire aux faces avant et arrière 4, et légèrement inclinée vers l'intérieur du connecteur. L'angle entre les faces supérieure 22 et inférieure 26 de l'échancrure sera par exemple compris entre 2° et 10°. Les échancrures 14 délimitent avec le conduit 12 s'étendant entre elles, un logement 28 pour la réception d'une traverse comme on le verra. De plus, de part et d'autre de l'axe du conduit 12, les échancrures 14 délimitent deux jonctions 30 entre lesquelles s'étend le conduit 12 et le logement 28.

Une partie du connecteur s'étendant au-dessus des jonctions 30 forme une tête 32. Sur la tête 32, les deux faces latérales 20 sont formées comme deux secteurs d'un même cylindre coaxial au conduit 12.

Le connecteur comporte une face cylindrique inférieure 34 contiguë à la face inférieure 10 du connecteur, perpendiculaire au conduit 12 et à mi-distance des deux faces latérales 20. Les échancrures 14 et cette face cylindrique 34 délimitent deux mors 36 reliés à la tête 32 chacun par les deux jonctions 30. Sur chaque jonction 30, le connecteur présente une fente 38 s'étendant en direction opposée aux mors 36, vers la tête 32.

Le conduit 12 présente un diamètre plus petit au niveau de la tête 32 que dans le reste du connecteur. Dans la tête 32, ce conduit 12 présente un filet 40.

En référence à la figure 2, si l'on dénomme « largeur » la dimension mesurée perpendiculairement aux faces latérales 20 du connecteur, la largeur j des jonctions 30 est inférieure à la largeur t de la tête 32 et à la largeur c du connecteur au niveau des mors 36.

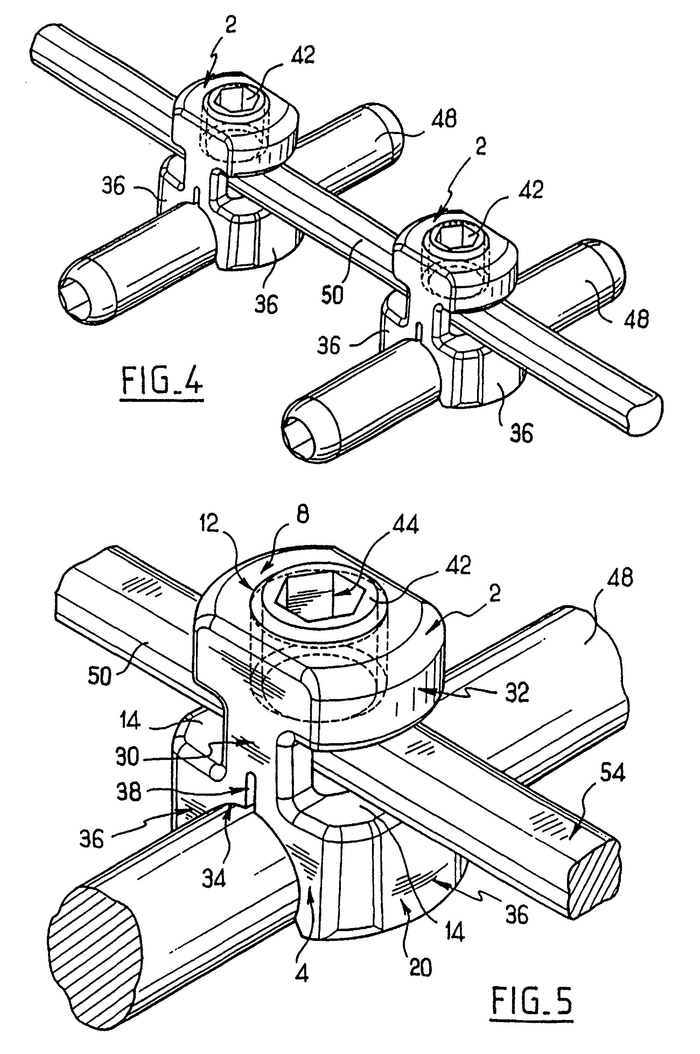

On va maintenant décrire le dispositif d'ostéosynthèse en référence aux figures 4 à 7.

Le dispositif comporte au moins deux connecteurs 2 tels que celui de la figure 1. Il comporte deux vis 42 adaptées à former une liaison vis-écrou en s'engageant dans le conduit 12 de la tête 32 des connecteurs respectifs. Chaque vis 42 présente une empreinte hexagonale 44 pour la réception d'une clé à six pans afin de manoeuvrer la vis 42 reçue dans le conduit 12. Le dispositif comprend deux tiges longitudinales rectilignes 48 destinées à s'étendre le long du rachis d'un patient en étant fixées chacune aux vertèbres par des organes d'ancrage non représentés et connus en eux-mêmes. Les deux tiges 48 ont un profil circulaire de même diamètre que la face cylindrique 34 des mors. Les mors 36 sont adaptés à réaliser un contact surface contre surface avec cette tige. La face cylindrique 34 des mors s'étend au total sur un arc de cercle supérieur à 180° et choisi en fonction des propriétés du matériau du connecteur de sorte qu'on peut engager les mors 36 sur la tige 48 en les clipsant sur celle-ci. La face cylindrique 34 des mors a un contour géométrique représenté en tirets sur la figure 2 qui s'étend au-delà de la face inférieure 10 du connecteur 2. Ainsi, lorsque la tige 48 est engagée entre les mors 36, la tige s'étend en saillie des mors suivant une direction radiale à la tige.

Le dispositif comporte enfin une traverse rectiligne 50 à section généralement rectangulaire adaptée à s'étendre dans les logements 28 en traversant les connecteurs 2 de part en part.

Tous les éléments du dispositif sont en acier inoxydable de préférence biocompatible.

Le montage du dispositif s'effectue comme suit.

On commence par clipser les connecteurs 2 sur les tiges longitudinales 48 respectives. Puis on engage la traverse 50 dans les deux logements 28 des connecteurs. On introduit les vis 42 dans les conduits 12 des connecteurs. Après qu'une position convenable a été atteinte pour les deux tiges 48 et la traverse 50, on serre chacune des vis 42.

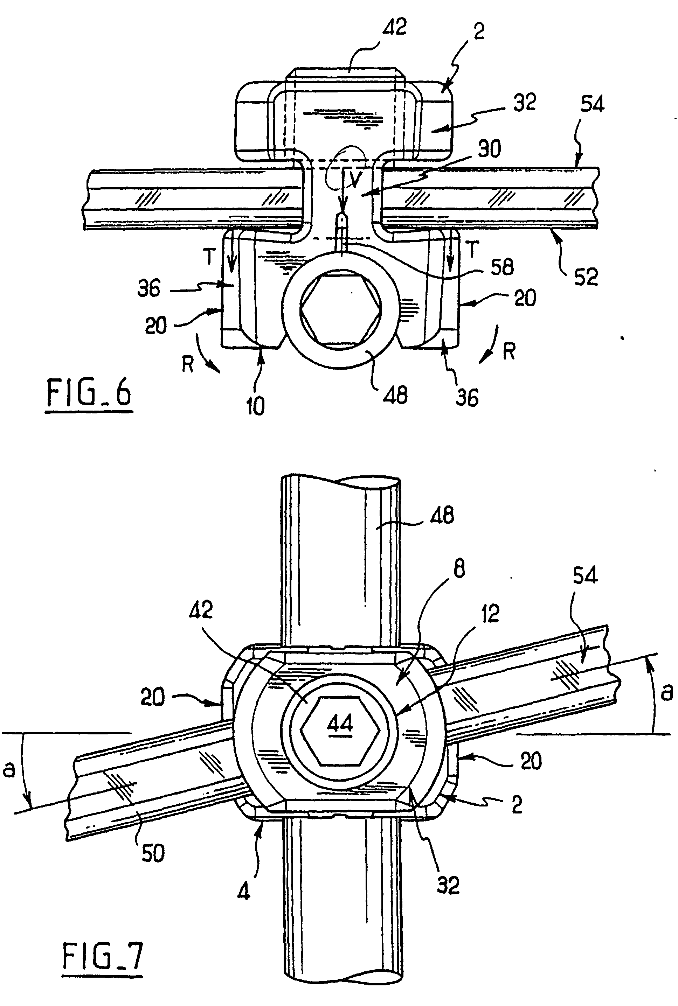

Comme on le voit notamment sur la figure 6, une face plane inférieure 52 de la traverse 50 vient alors en appui sur les mors 36, en étant au contact de ceux-ci uniquement aux bords 54 constituant l'intersection entre la face latérale 20 et la face inférieure inclinée 26 de l'échancrure 14, du fait de l'inclinaison de cette face 26 vers l'intérieur du connecteur.

Lors du serrage, la vis 42 sollicite la traverse 50 vers le bas en direction des mors 36 suivant l'axe du conduit 12 sur une face supérieure plane 54 de cette tige. Cette sollicitation est représentée par la flèche V. Cette sollicitation V entraîne que la tige 52 sollicite les deux mors 36 au niveau de leurs arêtes 54 dans la même direction et dans le même sens. Les sollicitations correspondantes sont représentées par les flèches T. Du fait de la configuration des jonctions 30, des fentes 38 et de l'élasticité du matériau, les mors 36 sont mobiles à rotation par rapport à la tête 32 autour d'un axe 58 parallèle à la tige longitudinale 48 et passant par les fentes 38. Cette mobilité est représentée par les flèches R. Dès lors, les sollicitations T, distances de l'axe 58 et perpendiculaires à celui-ci, déplacent les mors 36 en rotation en direction l'un de l'autre pour le serrage de la tige longitudinale 48.

Le logement 28 est configuré de sorte qu'il autorise un débattement angulaire a de la traverse 50 autour de l'axe du conduit 12, de part et d'autre de la direction perpendiculaire à la tige 48, comme le montre la figure 7. On peut ainsi fixer la traverse 50 dans une position non exactement perpendiculaire à la tige 48. Le débattement a sera par exemple de plus ou moins 15° de part et d'autre de la direction perpendiculaire, soit 30° au total.

On a représenté seulement une paire de connecteurs 2 et une traverse 50 associés aux tiges longitudinales 48. Toutefois, le dispositif d'ostéosynthèse comprendra avantageusement plusieurs paires de connecteurs 2 ayant chacune une traverse 50, associés à ces mêmes deux tiges longitudinales 48 le long du rachis.

On pourra prévoir que les mors 36 sont adaptés de par l'élasticité du matériau du connecteur à serrer la tige 48 en l'absence de toute sollicitation par la traverse et la vis 42. Il suffit pour cela de former les mors dans une position naturellement plus proche l'un de l'autre que lorsqu'ils reçoivent la tige 48. Néanmoins, les mors autoriseront un déplacement manuel du connecteur le long de la tige pour son repositionnement.

Alternativement, on pourra prévoir qu'en l'absence de serrage par la traverse 50 et la vis 42, le connecteur 2 est adapté à glisser sur la tige 48.

## Revendications

1. Connecteur (2) pour dispositif d'ostéosynthèse du rachis, comportant deux mors (36) et présentant un logement (28) adapté à recevoir une tige (50) prenant appui sur les mors (36), le connecteur (2) étant agencé de sorte qu'une sollicitation sur la tige (50) reçue dans le logement, en direction des mors (36), sollicite les mors en direction l'un de l'autre, **caractérisé en ce que** le connecteur (2) est d'une seule pièce.

2. Connecteur selon la revendication 1, **caractérisé en ce qu'**il comporte au moins une jonction (30) contiguë aux mors (36), la jonction ayant une largeur (j) inférieure à une largeur (c) du connecteur au niveau des mors.

3. Connecteur selon la revendication 2, **caractérisé en ce que** la jonction (30) présente une fente (38) s'étendant à partir des mors (36) en direction opposée à ceux-ci.

4. Connecteur selon la revendication 2 ou 3, **caractérisé en ce qu'**il comporte une tête (32) reliée aux mors (36) par la jonction (30), la largeur (j) de la jonction étant inférieure à une largeur (t) de la tête.

5. Connecteur selon l'une quelconque des revendications 2 à 4, **caractérisé en ce qu'**il est agencé de sorte que la tige (50) reçue dans le logement (28) est en appui sur une zone (54) des mors (36) distante de la jonction (30).

6. Connecteur selon la revendication 5, **caractérisé en ce qu'**il est agencé de sorte que la tige (50) reçue dans le logement (28) est en appui sur un bord (54) des mors (36) distant de la jonction (30).

7. Connecteur selon la revendication 6, **caractérisé en ce que** les mors (36) présentent chacun une face plane (26) adaptée à s'étendre en regard de la tige (50) recue dans le logement (28), cette face étant inclinée vers l'intérieur du connecteur.

8. Connecteur selon l'une quelconque des revendications 1 à 7, **caractérisé en ce qu'**il présente une face filetée (12), le logement (28) s'étendant entre les mors (36) et la face filetée.

9. Connecteur selon la revendication 8, **caractérisé en ce que** la face filetée (12) est formée par un conduit s'étendant dans le connecteur.

10. Dispositif d'ostéosynthèse comportant des première et deuxième tiges (48, 50), un organe fileté (42) et un connecteur (2) selon l'une quelconque des revendications précédentes dont les deux mors (36) sont adaptés à serrer la première tige (48), le connecteur présentant une face filetée (12) et le logement (28) s'étendant entre les mors (36) et la face filetée (12) et étant adapté à recevoir la deuxième tige (50) prenant appui sur les mors, le connecteur étant agencé de sorte qu'une sollicitation (T) par l'organe fileté (42) coopérant avec la face filetée (12), sur la deuxième tige (50) reçue dans le logement (28), en direction des mors (36), sollicite les mors en direction l'un de l'autre pour le serrage de la première tige (48).

11. Dispositif selon la revendication 10, **caractérisé en ce que** les mors (36) sont adaptés à être clipsés sur la première tige (48).

12. Dispositif selon la revendication 11, **caractérisé en ce que** les mors (36) sont adaptés à serrer la première tige (48) en l'absence d'une sollicitation sur les mors.

13. Dispositif selon l'une quelconque des revendications 10 à 12, **caractérisé en ce que** les mors (36) sont configurés de sorte que la première tige (48) s'étend en saillie des mors suivant une direction radiale à la première tige, lorsque la première tige est reçue entre les mors.

14. Dispositif selon l'une quelconque des revendications 10 à 13, **caractérisé en ce que** le connecteur (2) est agencé de sorte que la deuxième tige (50) peut être reçue dans le logement (28) en ayant une position angulaire variable par rapport à une direction perpendiculaire à la première tige (48).

15. Dispositif selon l'une quelconque des revendications 10 à 14, **caractérisé en ce que** la première tige (48) forme une liaison intervertébrale le long d'un rachis, et la deuxième tige (50) forme une union transversale entre la première tige (48) et une autre tige (48) formant une liaison intervertébrale.

## Claims

1. A connector (2) for a backbone osteosynthesis device, the connector having two jaws (36) and presenting a housing (28) adapted to receive a rod (50) bearing against the jaws (36), the connector (2) being arranged so that force on the rod (50) received in the housing and acting towards the jaws (36) urges the jaws towards each other, the connector (2) being **characterized in that** it is made as a single piece.

2. A connector according to claim 1, **characterized in that** it includes at least one junction (30) contiguous with the jaws (36), the junction being of a width (j) that is smaller than the width (c) of the connector level with the jaws.

3. A connector according to claim 2, **characterized in that** the junction (30) has a notch (38) extending from the jaws (36) in the opposite direction to the jaws.

4. A connector according to claim 2 or 3, **characterized in that** it has a head (32) connected to the jaws (36) by the junction (30), the width (j) of the junction being less than the width (t) of the head.

5. A connector according to any one of claims 2 to 4, **characterized in that** it is arranged in such a manner that the rod (50) received in the housing (28) bears against respective zones (54) of the jaws (36) that are remote from the junction (30).

6. A connector according to claim 5, **characterized in that** it is arranged in such a manner that the rod (50) received in the housing (28) bears against respective edges (54) of the jaws (36) that are remote from the junction (30).

7. A connector according to claim 6, **characterized in that** each of the jaws (36) presents a plane face (26) adapted to extend facing the rod (50) received in the housing (28), said face sloping towards the inside of the connector.

8. A connector according to any one of claims 1 to 7, **characterized in that** it has a threaded face (12), the housing (28) extending between the jaws (36) and the threaded face.

9. A connector according to claim 8, **characterized in that** the threaded face (12) is formed by a duct extending inside the connector.

10. An osteosynthesis device comprising first and second rods (48, 50), a threaded member (42), and a connector (2) according to any preceding claim in which the two jaws (36) are adapted to clamp onto the first rod (48), the connector having a threaded face (12) and the housing (28) extending between the jaws (36) and the threaded face (12) and being adapted to receive the second rod (50) bearing against the jaws, the connector being arranged so that a force (T) applied by the threaded member (42) cooperating with the threaded face (12), against the second rod (50) received in the housing (28) towards the jaws (36) urges the jaws towards each other so as to clamp onto the first rod (48).

11. A device according to claim 10, **characterized in that** the jaws (36) are adapted to be dipped onto the first rod (48).

12. A device according to claim 11, **characterized in that** the jaws (36) are adapted to clamp onto the first rod (48) in the absence of any force being applied to the jaws.

13. A device according to any one of claims 10 to 12, **characterized in that** the jaws (36) are configured in such a manner that the first rod (48) projects from the jaws in a radial direction of the first rod when the first rod is received between the jaws.

14. A device according to any one of claims 10 to 13, **characterized in that** the connector (2) is arranged so that the second rod (50) can be received in the housing (28) while occupying a range of angular positions about a direction perpendicular to the first rod (48).

15. A device according to any one of claims 10 to 14, **characterized in that** the first rod (48) forms a link between vertebrae along a backbone, and the second rod (50) forms a transverse union between the first rod (48) and another rod (48) forming a link between vertebrae.

## Patentansprüche

1. Verbindungsstück (2) für eine Osteosynthesevorrichtung des Rückgrates, mit zwei Spannbacken (36), und eine Aufnahme (28), die dafür eingerichtet ist, eine Stange (50) aufzunehmen, welche sich auf die Spannbacken (36) auflegt, wobei das Verbindungsstück (2) derart eingerichtet ist, dass eine Beanspruchung der in der Aufnahme aufgenommenen Stange (50) in Richtung der Spannbacken (36) die Spannbacken in Richtung zueinander hin spannt, **dadurch gekennzeichnet, dass** das Verbindungsstück (2) aus einem einzigen Stück gebildet ist.

2. Verbindungsstück gemäß Anspruch 1, **dadurch gekennzeichnet, dass** es wenigstens eine an die Spannbacken (36) angrenzende Verbindungsstelle (30) aufweist, wobei die Verbindungsstelle eine kleinere Breite (j) als eine Breite (c) des Verbindungsstückes auf der Höhe der Spannbacken aufweist.

3. Verbindungsstück gemäß Anspruch 2, **dadurch gekennzeichnet, dass** die Verbindungsstelle (30) eine Spalte (38) aufweist, die sich ausgehend von den Spannbacken (36) in deren entgegen gesetzte Richtung erstreckt.

4. Verbindungsstück gemäß Anspruch 2 oder 3, **dadurch gekennzeichnet, dass** es einen Kopf (32) aufweist, der durch die Verbindungsstelle (30) mit den Spannbacken (36) verbunden ist, wobei die Breite (j) der Verbindungsstelle kleiner als eine Breite (t) des Kopfes ist.

5. Verbindungsstück gemäß irgendeinem der Ansprüche 2 bis 4, **dadurch gekennzeichnet, dass** es derart angeordnet ist, dass die in der Aufnahme (28) aufgenommene Stange (50) auf einem von der Verbindungsstelle (30) entfernt liegenden Bereich (54) der Spannbacken (36) aufliegt.

6. Verbindungsstück gemäß Anspruch 5, **dadurch gekennzeichnet, dass** es derart angeordnet ist, dass die in der Aufnahme (28) aufgenommene Stange (50) auf einer von der Verbindungsstelle (30) entfernt liegenden Kante (54) der Spannbacken (36) aufliegt.

7. Verbindungsstück gemäß Anspruch 6, **dadurch gekennzeichnet, dass** die Spannbacken (36) jeweils eine ebene Fläche (26) aufweisen, die dafür eingerichtet ist, sich gegenüber der in der Aufnahme (28) aufgenommenen Stange (50) zu erstrecken, wobei diese Fläche zu dem inneren Bereich des Verbindungsstückes hin geneigt ist.

8. Verbindungsstück gemäß irgendeinem der Ansprüche 1 bis 7, **dadurch gekennzeichnet, dass** es eine gewindete Fläche (12) aufweist, wobei sich die Aufnahme (28) zwischen den Spannbacken (36) und der gewindeten Fläche erstreckt.

9. Verbindungsstück gemäß Anspruch 8, **dadurch gekennzeichnet, dass** die gewindete Fläche (12) von einem sich in das Verbindungsstück erstreckenden Kanal gebildet ist.

10. Osteosynthesevorrichtung mit einer ersten und zweiten Stange (48, 50), einem gewindeten Organ (42) und einem Verbindungsstück (2) gemäß irgendeinem der vorherigen Ansprüche, dessen zwei Spannbacken (36) dafür eingerichtet sind, die erste Stange (48) einzuspannen, wobei das Verbindungsstück eine gewindete Fläche (12) aufweist und wobei die Aufnahme (28), sich zwischen den Spannbacken (36) und der gewindeten Fläche (12) erstreckt, und welche dafür eingerichtet ist, die zweite Stange (50) aufzunehmen, welche sich auf die Spannbacken auflegt, wobei das Verbindungsstück derart angeordnet ist, dass eine Beanspruchung (T) der zweiten, in der Aufnahme (28) aufgenommenen Stange (50) in Richtung der Spannbacken (36) durch das gewindete Organ (42), das mit der gewindeten Fläche (12) zusammenwirkt, die Spannbacken in Richtung zueinander hin spannt, um die erste Stange (48) einzuspannen.

11. Vorrichtung gemäß Anspruch 10, **dadurch gekennzeichnet, dass** die Spannbacken (36) dafür eingerichtet sind, auf der ersten Stange (48) festgeklemmt zu werden.

12. Vorrichtung gemäß Anspruch 11, **dadurch gekennzeichnet, dass** die Spannbacken (36) dafür eingerichtet sind, die erste Stange (48) bei Fehlen einer Spannung auf den Spannbacken einzuspannen.

13. Vorrichtung gemäß irgendeinem der Ansprüche 10 bis 12, **dadurch gekennzeichnet, dass** die Spannbacken (36) derart gestaltet sind, dass die erste Stange (48) von den Spannbacken gemäß einer radialen Richtung zu der ersten Stange vorspringt, wenn die erste Stange zwischen den Spannbacken aufgenommen ist.

14. Vorrichtung gemäß irgendeinem der Ansprüche 10 bis 13, **dadurch gekennzeichnet, dass** das Verbindungsstück (2) derart angeordnet ist, dass die zweite Stange (50) in der Aufnahme (28) aufgenommen werden kann, wobei sie eine bezüglich einer senkrecht zu der ersten Stange (48) liegenden Richtung variable Winkelposition einnimmt.

15. Vorrichtung gemäß irgendeinem der Ansprüche 10 bis 14, **dadurch gekennzeichnet, dass** die erste Stange (48) eine intervertebrale Verbindung entlang des Rückgrates bildet, und die zweite Stange (50) eine Querverbindung zwischen der ersten Stange (48) und einer anderen, eine intervertebrale Verbindung bildenden Stange (48) bildet.
